# EUROPEAN PATENT SPECIFICATION

(11) **EP 3 326 596 B1**
(45) Date of publication and mention of the grant of the patent: **23.12.2020**
(21) Application number: 17203877.0
(22) Date of filing: 27.11.2017
(51) Int. Cl.: A61F 13/49, A61F 13/496, A61F 13/15

(54) **METHOD OF MAKING WEARABLE ARTICLE COMPRISING ELASTIC BELT**
VERFAHREN ZUR HERSTELLUNG EINES WEARABLE-ARTIKELS MIT ELASTISCHEM BAND
PROCÉDÉ DE FABRICATION D'UN ARTICLE VESTIMENTAIRE COMPRENANT UNE CEINTURE ÉLASTIQUE

(30) Priority: 25.11.2016 WO PCT/IB2016/107182
(43) Date of publication of application: 30.05.2018
(73) Proprietor: The Procter & Gamble Company, Cincinnati, OH 45202 (US)
(72) Inventor: TAMEISHI, Kazuaki, Akashi, Hyogo 674-0093 (JP); LEE, DonSub, Akashi, Hyogo 674-0093 (JP); YAMASHITA, Fumitake, Akashi, Hyogo 674-0093 (JP); JIA, Ying, Beijing, 101312 (CN)
(74) Representative: P&G Patent Germany

(56) References cited:
- WO-A1-2016/029566
- US-A1- 2006 030 831
- US-A1- 2013 211 357
- US-A1- 2013 317 471

## Description

### FIELD OF THE INVENTION

The present invention relates to a method of making a wearable article comprising an elastic belt, and articles made thereof.

### BACKGROUND OF THE INVENTION

Infants and other incontinent individuals wear absorbent articles such as diapers to receive and contain urine and other body exudates. Pull-on absorbent articles, or pant-type absorbent articles, are those which are donned by inserting the wearer's legs into the leg openings and sliding the article up into position about the lower torso. Pant-type absorbent articles have become popular for use on children who are able to walk and often who are toilet training, as well as for younger children who become more active in movement such that application of taped-type absorbent articles tends to be more difficult.

Belt-type pants having a main body to cover the crotch region of the wearer and a separate elastic belt defining the waist opening and leg opening are known in the art, such as described in PCT Publication WO 2006/17718A. Such belt-type pants have an elastic belt made of a laminate of 2 substrate layers and elastic bodies sandwiched therebetween. These elastic belts may be economically made by joining the elastic bodies to one of such substrate layers, overlaying and joining the other substrate layer on top to make a laminate, and then deactivating the elastic bodies in certain regions to, for example, avoid having elasticity where the elastic belt would overlap the absorbent core. While such method of making the elastic belt may be conveniently conducted by joining and deactivating all of the elastic bodies of the laminate concurrently, this may not be effective or provide the best results when different force or tactile sense is desired for different portions of the belt. Further, when the laminate is made of 2 layers of substrates, one of the layers may be folded over to avoid having sharp edges at the waist opening or the leg opening. Such a configuration results in regions that are provided in 3 layers and 2 layers. The resulting difference in layers may provide disadvantages such as non-uniform gathering to the laminate, less controllable force profile, or undesirable tactile sense. There may be interest to decrease or eliminate such difference of layers, or to provide a construction wherein the differing layers provide specific functions.

Based on the foregoing, there is a need for a method of making a wearable article comprising an elastic belt with various belt configurations in an effective and economical manner.

### SUMMARY OF THE INVENTION

The present invention is directed to a method of manufacturing a wearable article assembled by a front belt, a back belt, and a central chassis, the central chassis having a front waist region and a back waist region separated from each other by a crotch region; wherein at least one of the front belt and the back belt is formed by an elastic belt,
the method comprising making the elastic belt and joining to the central chassis by the steps of;
advancing a first layer of continuous sheet having a first surface and an opposing second surface in a machine direction and defining a width in a cross machine direction;
advancing a second layer of continuous sheet having a first surface and an opposing second surface in the machine direction and having a smaller width than the first layer of continuous sheet;
advancing a first group of elastic bodies in the machine direction in a stretched state;
joining the first group of elastic bodies between the first surface of the first layer and the first surface of the second layer of continuous sheets to form a primary elastic belt precursor
advancing a second group of elastic bodies in the machine direction in a stretched state;
joining the second group of elastic bodies to the first surface of the first layer of continuous sheet of the primary elastic belt precursor;
joining the central chassis to the second surface of the second layer of continuous sheet;
folding the first layer of continuous sheet to form a first layer fold over of continuous sheet, such that at least one of the second group of elastic bodies is directly joined between the first surface of the first layer of continuous sheet and the first surface of the first layer fold over of continuous sheet.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:
Figure 1 is a perspective view of one embodiment of a wearable article of the present invention.
Figure 2 is a schematic plan view of one embodiment of a wearable article of the present invention with the seams unjoined and in a flat uncontracted condition showing the garment facing surface.
Figure 3A is a schematic cross section view of Figure 2 taken along line L1 in one embodiment of belt structure, either on the front belt or the back belt.
Figure 3B is a schematic cross section view of Figure 2 taken along line L2 in one embodiment of belt structure, either on the front belt or the back belt.
Figure 3C is a schematic cross section view of Figure 2 taken along line L3 in one embodiment of belt structure, either on the front belt or the back belt.
Figure 4 is a schematic cross section view of a wearable article of the prior art.
Figure 5-7 are schematic cross section views of Figure 2 taken along line L2 in other embodiments of belt structure, either on the front belt or the back belt.
Figure 8 is a schematic view of the process of the present invention.
Figure 9 is a schematic view of the primary elastic belt precursor forming step of the process of the present invention.
Figure 10 is a schematic view of the second elastic introduction step of the process of the present invention.
Figure 11 is a schematic view of the central chassis joining step of the process of the present invention.
Figure 12 is a schematic view of the central chassis forming and joining step of the process of the present invention.
Figure 13 is a schematic view of the first layer folding step of the process of the present invention.
Figure 14 is a schematic view of the central chassis folding, side seaming, and final cut steps of the process of the present invention.
Figure 15 is a schematic view of the third elastic bodies joining step of the process of the present invention.
Figure 16 is a schematic view of another process of the present invention.

### DEFINITIONS

As used herein, the following terms shall have the meaning specified thereafter:
"Wearable article" refers to articles of wear which may be in the form of pants, taped diapers, incontinent briefs, feminine hygiene garments, and the like. The "wearable article" may be so configured to also absorb and contain various exudates such as urine, feces, and menses discharged from the body. The "wearable article" may serve as an outer cover adaptable to be joined with a separable disposable absorbent insert for providing absorbent and containment function, such as those disclosed in PCT publication WO 2011/087503A.
"Pant" refers to disposable absorbent articles having a pre-formed waist and leg openings. A pant may be donned by inserting a wearer's legs into the leg openings and sliding the pant into position about the wearer's lower torso. Pants are also commonly referred to as "closed diapers", "prefastened diapers", "pull-on diapers", "training pants" and "diaper-pants".
"Longitudinal" refers to a direction running substantially perpendicular from a waist edge to an opposing waist edge of the article and generally parallel to the maximum linear dimension of the article. "Transverse" refers to a direction perpendicular to the longitudinal direction.
"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).
"Disposed" refers to an element being located in a particular place or position.
"Joined" refers to configurations whereby an element is directly secured to another element by affixing the element directly to the other element and to configurations whereby an element is indirectly secured to another element by affixing the element to intermediate member(s) which in turn are affixed to the other element. Joining may be provided by applying adhesive agents, ultrasound or by embossing the at least two elements to be affixed to one another.
"Proximal" refers to a portion being closer or planned to be closer relative to the longitudinal center of the article, while "distal" refers to a portion being farther or planned to be farther from the longitudinal center of the article.
"Film" refers to a sheet-like material wherein the length and width of the material far exceed the thickness of the material. Typically, films have a thickness of about 0.5 mm or less.
"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".
"Extendibility" and "extensible" mean that the width or length of the component in a relaxed state can be extended or increased.
"Elasticated" and "elasticized" mean that a component comprises at least a portion made of elastic material.
"Elongatable material", "extensible material", or "stretchable material" are used interchangeably and refer to a material that, upon application of a biasing force, can stretch to an elongated length of at least about 110% of its relaxed, original length (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastomeric." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "substantially non-elastic" or "substantially non-elastomeric". For example, an elongatable material that has an initial length of 100mm can extend at least to 150mm, and upon removal of the force retracts to a length of at least 145mm (i.e., exhibiting a 10% recovery).

### DETAILED DESCRIPTION OF THE INVENTION

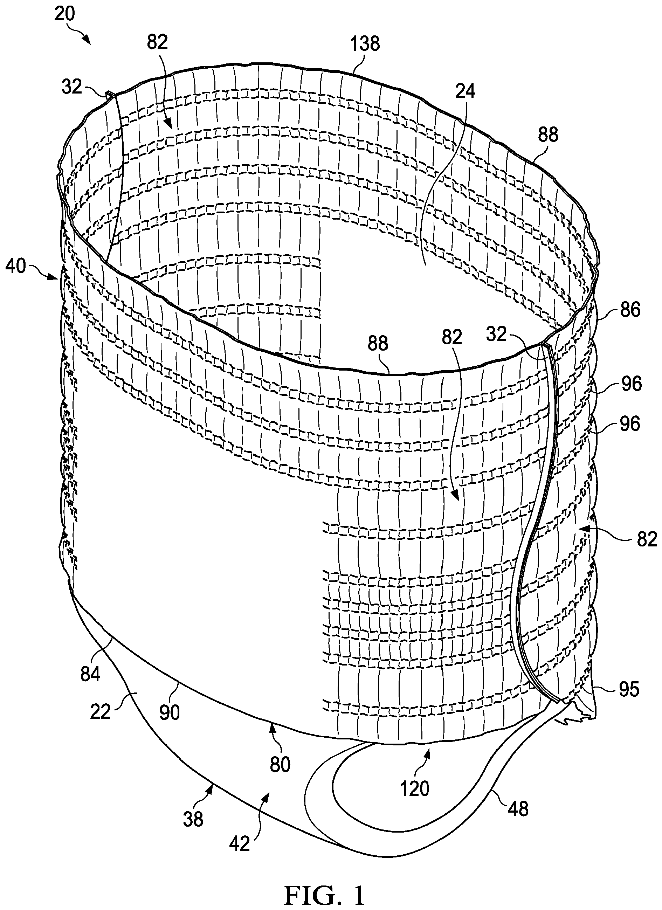

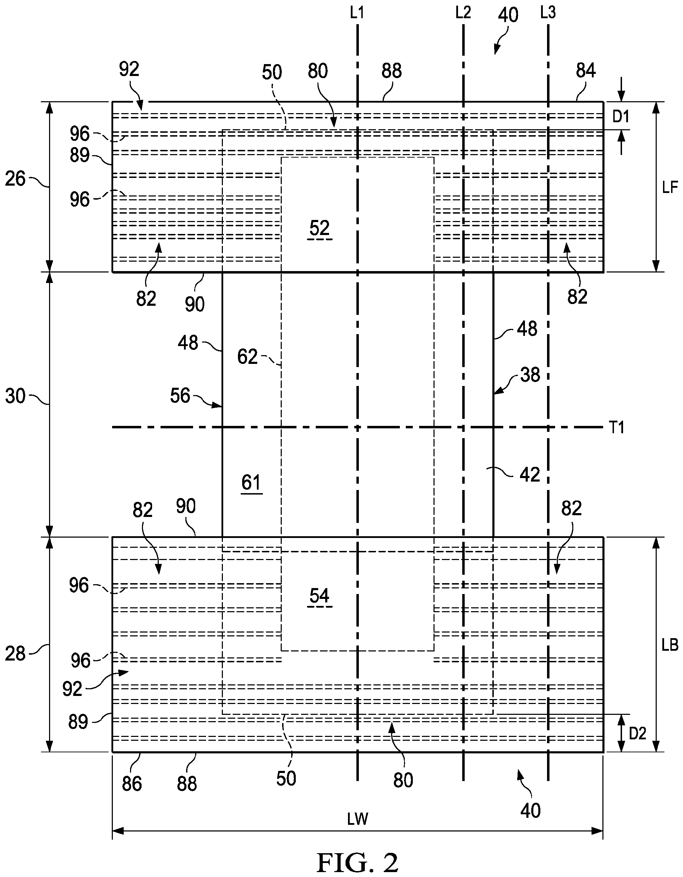

Figure 1 is a perspective view of an embodiment of the wearable article 20 made by the present invention and Figure 2 is a schematic plan view of the same article with the seams unjoined and in its flat uncontracted condition showing the garment-facing surface. In Figures 1 and 2, the position of the elastics 96 may or may not be accurate. The wearable article 20 has a longitudinal centerline L1 which also serves as the longitudinal axis, and a transverse centerline T1 which also serves as the transverse axis. The wearable article 20 has a body facing surface, a garment facing surface, a front region 26, a back region 28, a crotch region 30, and seams 32 which join the front region 26 and the back region 28 to form two leg openings and a waist opening. The wearable article 20 comprises a center chassis 38 to cover the crotch region of the wearer, a front belt 84 and a back belt 86 (hereinafter may be referred to as "front and back belts"), the front and back belts 84, 86 forming a ring-like elastic belt 40 (hereinafter may be referred to as "waist belt") extending transversely defining the waist opening. The front and back belts 84, 86 and the center chassis 38 jointly define the leg openings.

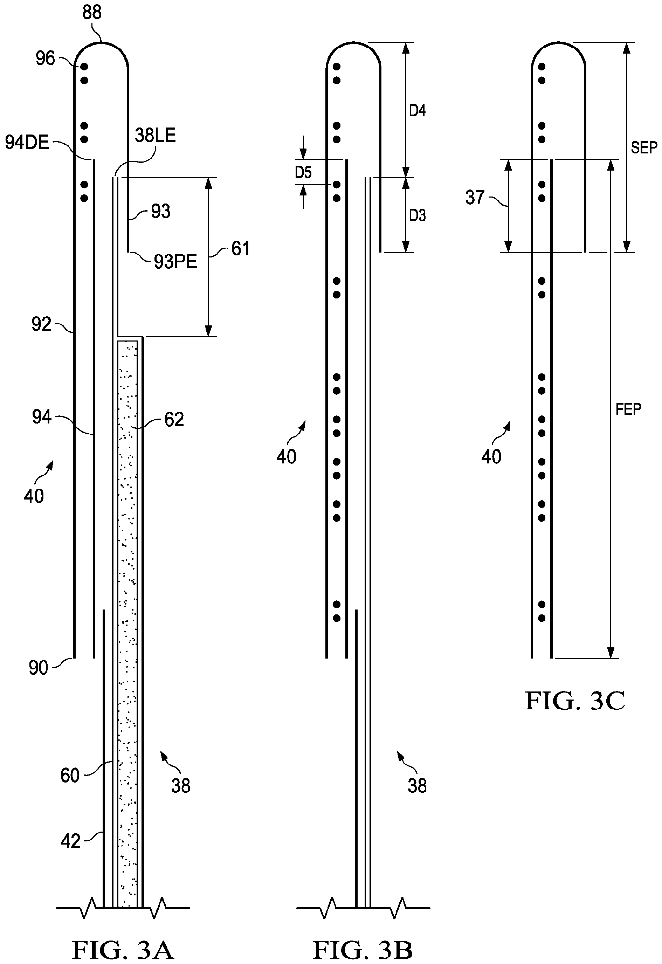

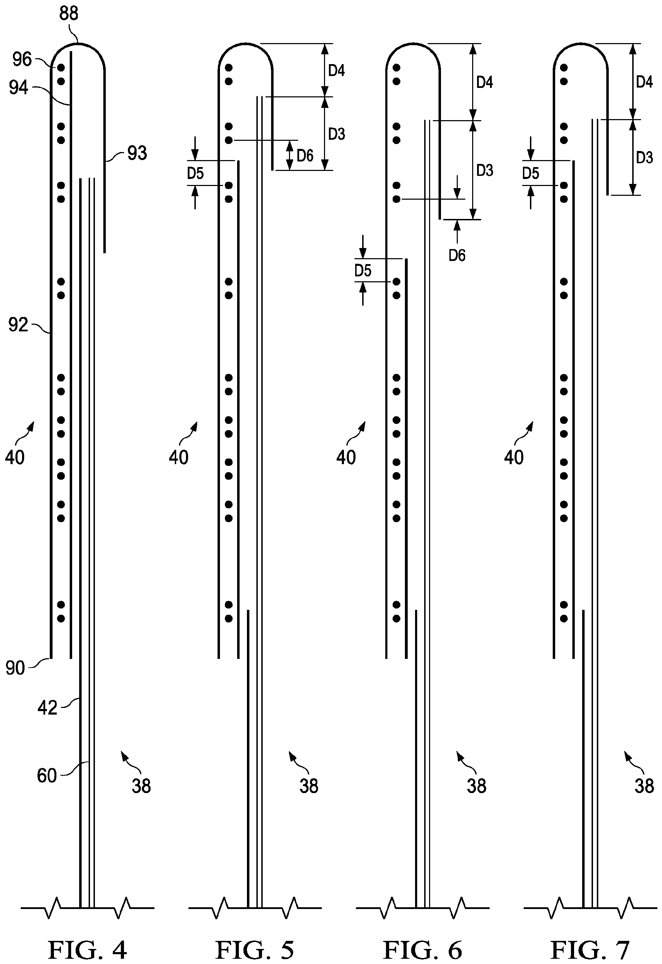

Figures 3A, 3B, and 3C are schematic cross section views of one embodiment article made by the present invention, taken along lines L1, L2, and L3, respectively. L1 describes the longitudinal centerline, while L2 describes a longitudinal line running through the transverse edge of the center chassis 38, and L3 describes a longitudinal line running through the left and right panels 82 where the center chassis 38 does not exist. Figure 4 is a schematic cross section view of the prior art taken along L2 of a belt-type pant article. Figure 5-7 is a schematic cross section view of other article embodiments made by the present invention taken along L2. In Figures 3A-C, and 4-7, the thickness dimension may be exploded and exaggerated.

Referring to Figure 3A, the center chassis 38 comprises a backsheet 60 and outer cover layer 42 for covering the garment-facing side of the backsheet 60. The backsheet 60 may be a water impermeable film. The outer cover layer 42 may be a nonwoven sheet. The center chassis 38 may contain an absorbent material existing region 62 for absorbing and containing body exudates disposed on the center chassis 38, and an absorbent material non-existing region 61 surrounding the periphery of the absorbent material existing region 62. In the embodiment shown in Figure 2, the center chassis 38 has a generally rectangular shape, left and right longitudinally extending side edges 48 (hereinafter may be referred to as "side edge") and front and back transversely extending end edges 50 (hereinafter may be referred to as "end edge"). The center chassis 38 also has a front waist panel 52 positioned in the front region 26 of the wearable article 20, a back waist panel 54 positioned in the back region 28, and a crotch panel 56 between the front and back waist panels 52, 54 in the crotch region 30. The center of the front belt 84 is joined to a front waist panel 52 of the center chassis 38, the center of the back belt 86 is joined to a back waist panel 54 of the center chassis 38, the front and back belts 84, 86 each having a left side panel and a right side panel 82 where the center chassis 38 does not overlap. The center chassis has a crotch panel 56 positioned between the front waist panel 52 and the back waist panel 54. The front and back belt are discontinuous of each other in the longitudinal direction.

Referring to Figures 1 and 2, the ring-like belt 40 formed by the front belt 84 and back belt 86 acts to dynamically create fitment forces and to distribute the forces dynamically generated during wear. The front and back belts 84, 86 may be joined with each other only at the side edges 89 at the seams 32 to form a wearable article having a waist opening and two leg openings. Each leg opening may be provided with elasticity around the perimeter of the leg opening by the combination of elasticity from the front belt 84, the back belt 86, and any from the center chassis 38. The front leg opening region 120 is disposed adjacent the leg opening along the proximal edge 90 of the left and right side panels 82 of the front belt 84.

The front belt 84 and back belt 86 are configured to impart elasticity to the belt 40. The front belt 84 and the back belt 86 may each be formed by a laminate comprising a plurality of elastic bodies 96 running in the transverse direction, an inner sheet 94, an outer sheet 92, and an outer sheet fold over 93 wherein the outer sheet fold over 93 is an extension of the outer sheet material formed by folding the outer sheet material at the distal edge 88 of the front and back belts; wherein the belt elastic bodies 96 are sandwiched between two of these sheets. The front belt 84 and the back belt 86 may each be made only by elastic bodies 96, the inner sheet 94, the outer sheet 92, and the outer sheet fold over 93. The outer sheet fold over 93 may be directly joined to the center chassis 38. The belt elastic bodies 96 may extend in the transverse direction to provide a ring like elastic belt 40 when the front belt 84 and the back belt 86 are joined. At least some of the elastic bodies 96 extend in the transverse direction substantially parallel to each other. All of the elastic bodies 96 may extend in the transverse direction substantially parallel to each other. Such an article may be economically made. The front and back belt 84, 86 each have transversely continuous proximal and distal edges, the proximal edge 90 being located closer than the distal edge 88 relative to the longitudinal center of the article. The outer sheet fold over 93 is an extension of the outer sheet material formed by folding the outer sheet material at the distal edge 88 of the front and back belts 84, 86. The elastic bodies 96 may be disposed in the same or different denier, interval, or force between the front and back, as well as in different longitudinal positions of the belt. The inner and outer sheets 92, 94 may be the same or different material, and selected to provide characteristics such as breathability, softness, cushiony feel, loftiness, and combinations thereof, depending on the desirables of the resulting article. The inner and outer sheets 92, 94 may have the same or different basis weight, stiffness, texture or any combination thereof.

Referring to Figure 2, the transverse width LW of the back belt 86 in the uncontracted condition may be the same as the transverse width of the front belt 84 of the same condition. Such an article may be economically made.

The longitudinal length LB of the back belt 86 between the back distal edge 88 and the back proximal edge 90 along its entire width LW of the back belt 86 may be approximately the same as the longitudinal length LF of the front belt 84 between the front distal edge 88 and the front proximal edge 90. In such configuration, the seams 32 close the front and back belt 84, 86 side edges 89 of the same length for forming the article. Such an article may be economically made.

The back belt 86 may have a greater longitudinal length LB between the back distal edge 88 and the back proximal edge 90 along its entire width LW of the back belt 86 in the transverse direction than the longitudinal length LF of the front belt 84 between the front distal edge 88 and the front proximal edge 90 (Figures 1 and 2). In such configuration, when the wearable article is assembled to form the waist opening and the leg openings, the wearable article 20 is folded along the transverse centerline T1 such that the front distal edge 88 is aligned with the back distal edge 88. The front side edge 89 is also aligned with a portion of the back side edge 89. Then the front belt 84 and the back belt 86 are joined at the front and back side edges 89 at the seams 32. The front and back proximal edges 90, however, may not be aligned to one another. The back proximal edge 90 may be disposed longitudinally closer than the front proximal edge 90 relative to the transverse center line T1 such that the proximal portion of the back side panel 82 extends toward the crotch panel 56 of the center chassis 38 beyond the front proximal edge 90. The side edge of the proximal portion of the back side panel 82 may not be joined to anywhere and free from attachment. Thus, the proximal portion of the back side panel 82 provides a buttock cover 95 as in Figure 1.

Articles known in the art such as in Figure 4 have the longitudinal length of the inner sheet approximately matching the longitudinal length of the elastic belt. This provides regions of the elastic belt having 3 layers for a significant percentage of the belt while leaving the other regions in 2 layers.

Referring to Figures 3A-C, the present invention enables manufacturing of articles having a region towards the distal edge of the elastic belt 88 wherein the elastic belt 40 is made only by the outer belt 92, the outer belt fold over 93, and elastics 96 disposed therebetween. In Figures 3B-C, there are 4 elastic bodies 96 which are disposed between the outer belt 92 and the outer belt fold over 93, 2 elastic bodies 96 which are disposed where 3 layers overlap (outer belt 92, inner belt 93, and outer belt fold over 93), and 12 elastic bodies 96 which are disposed between the inner belt 94 and the outer belt 92. Namely, a majority of elastic bodies are disposed between 2 layers.

Referring to Figures 5-6, the present invention also enables manufacturing articles which have all of the elastic bodies 96 disposed in only 2 layers, namely either between the outer belt 92 and the outer belt fold over 93, or between the inner belt 94 and the outer belt 92.

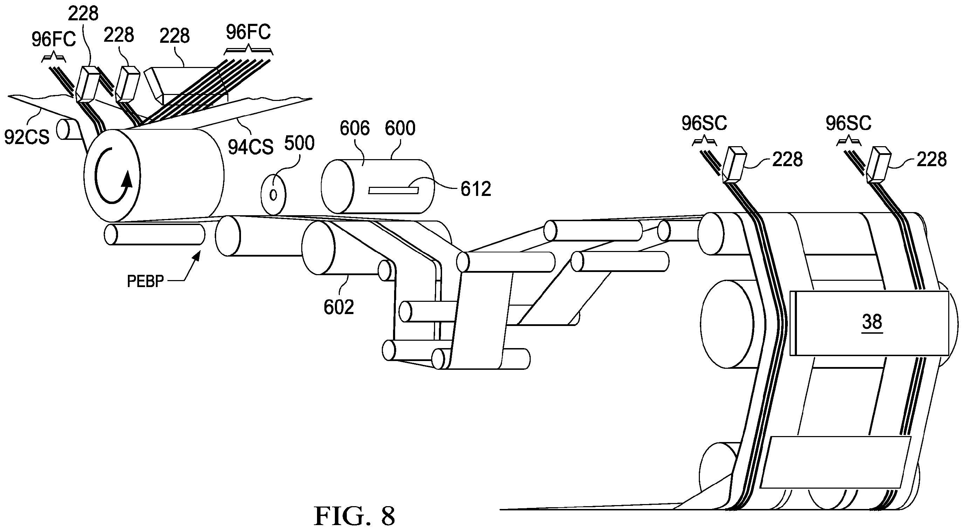

Referring to Figure 8, the present invention relates to making the elastic belt 40 and joining to the central chassis 38 by the following steps A) - D).
A) Related to forming a primary elastic belt precursor, or PEBP, the step comprising:
   advancing a first layer of continuous sheet 92CS having a first surface and an opposing second surface in a machine direction and defining a width in a cross machine direction;
   advancing a second layer of continuous sheet 94CS having a first surface and an opposing second surface in the machine direction and having a smaller width than the first layer of continuous sheet;
   advancing a first group of elastic bodies 96FC in the machine direction in a stretched state;
   joining the first group of elastic bodies 96FC between the first surface of the first layer and the first surface of the second layer of continuous sheets 92CS, 94CS, to form a PEBP.
B) Related to introducing the second group of elasic bodies 96SC comprising:
   advancing a second group of elastic bodies 96SC in the machine direction in a stretched state;
   joining the second group of elastic bodies 96SC to the first surface of the first layer of continuous sheet 92CS of the PEBP.
C) Related to joining the central chassis 38 to the second surface of the second layer of continuous sheet 94CS.
D) Related to folding the first layer to form a first layer fold over of continuous sheet 93CS, such that at least one of the second group of elastic bodies 96SC is directly joined between the first surface of the first layer of continuous sheet 92CS and the first surface of the first layer fold over of continuous sheet, 93CS.

In the finished article, the first layer of continuous sheet 92CS becomes the outer sheet 92 wherein the second surface of the first layer of continuous sheet 92CS becomes the garment facing surface, the second layer of continuous sheet 94CS becomes the inner sheet 94 wherein the second surface of the second layer of continuous sheet 94CS becomes the body facing surface, and the first layer fold over of continuous sheet 93CS becomes the outer sheet fold over 93 wherein the second surface of the first layer fold over of continuous sheet 93CS becomes the body facing surface.

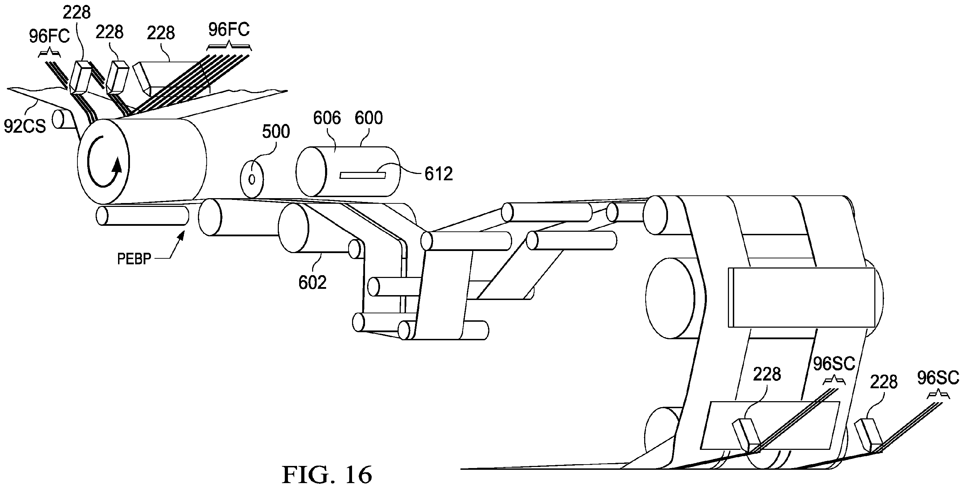

Figure 8 describes a schematic view of the entirety of the process of the present invention following the steps A) - D) in the order described above. Alternatively, steps B) and C) may be reversed, namely, the central chassis 38 may be joined to the PEBP prior to introducing the second group of elastic bodies 96SC on the proviso that, as seen in Figures 3A-C, there are no second group of elastic bodies 96SC planned to be disposed between the first layer of continuous sheet 93CS and the central chassis 38. Such alternative process is described in Figure 16.

### Step A)

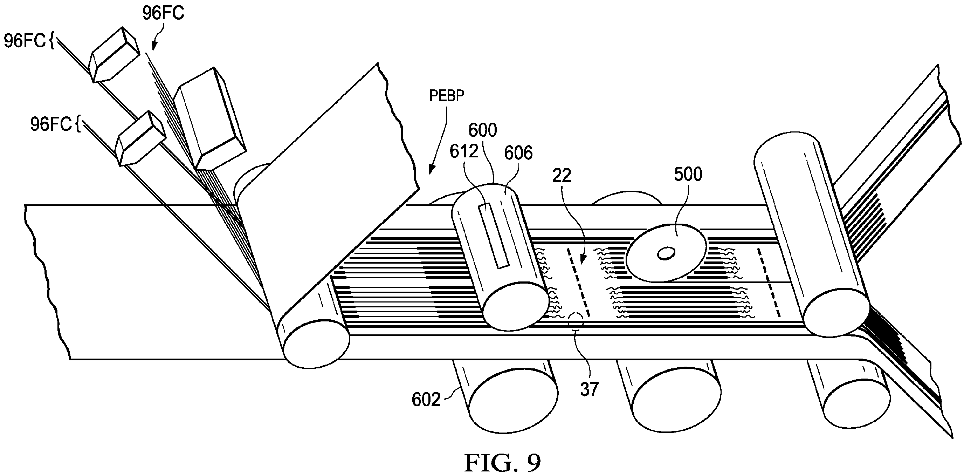

Referring to Figures 8 and 9, the PEBP forming step comprises advancing a first layer of continuous sheet 92CS having a first surface and an opposing second surface in a machine direction; advancing a second layer of continuous sheet 94CS having a first surface and an opposing second surface in the machine direction; advancing a first group of elastic bodies 96FC in the machine direction in a stretched state; and joining the first group of elastic bodies 96FC between the first surface of the first layer and the first surface of the second layers of continuous sheets 92CS, 94CS, to form a PEBP. The portion of the PEBP at which the first group of elastic bodies 96FC are joined may be referred to as the first elastic portion, or FEP.

In Figure 9 the first group of elastic bodies 96FC are placed in the center in the cross machine direction of the first layer of continuous sheet 92CS on the proviso that the PEBP will later be divided into 2 continuous belt precursor parts for making the front belt and back belt, wherein one or both of the belts may be a PEBP made by the present process. In Figure 9, the depictions after joining of the second layer of continuous sheet 94CS is shown as if the second layer of continuous sheet 94CS is removed and therefore the elastic bodies can be directly observed. The first and second layer of continuous sheets 94CS, 92CS have a width in a cross machine direction; wherein the second layer of continuous sheet 94CS has a smaller width than the first layer of continuous sheet 92CS to enable introducing the second group of elastic bodies 96SC in the later steps. The dimension of the second layer of continuous sheet 94CS and the positioning of first group of elastic bodies 96FC may be selected such that the elastic body closest to the edge of the second layer of continuous sheet 94CS is disposed at a distance D5 away from the edge of second layer of continuous sheet 94CS, wherein D5 may be at least about 5mm. Referring to Figures 3B-C and 5-7, this may prevent any elastic body 96 from being accidentally left uncovered by the inner sheet 94 in the finished article. The elastic bodies 96 may be joined between the first and second layers of continuous sheets 92CS, 94CS by any means known in the art, including use of adhesive material dispensed by adhesive dispensers 228 immediately before joining.

The obtained PEBP may be divided by a slitter 500 in the machine direction to form 2 continuous parts, which are the front belt and the back belt precursors, wherein either or both of the front and back belt precursors are the PEBP according to the present invention. The dividing may be provided before or after making of the elastic region and non-elastic region 22 as described below.

Whether or not the front and back belt take the configurations of the present invention, the front belt precursor and the back belt precursor may be different in at least one of; the dimension of the precursor, the number of first group of elastic bodies, and the positioning of the first group of elastic bodies.

Referring to Figure 8, the thus obtained FEP comprising: the lamination of the first and second layers of continuous sheets 92CS, 94CS and first group of elastic bodies 96FC; may comprise an elastic region and a non-elastic region 22. By elastic region, what is meant is a region which contributes to the elasticity of the waist belt 40 in the final article. By non-elastic region 22, what is meant is a region which does not contribute to the elasticity of the waist belt 40 in the final article. An elastic body unadhered to either of the first and second layers of continuous sheets 92CS, 94CS and cut in the unadhered area may be left dangling, thereby still exhibiting elasticity as an elastic body per se. However, so long as the elasticity is non-contributory to elasticity of the waist belt 40, such area is considered a non-elastic region 22. On the other hand, an elastic body unadhered to either or both of the first and second layers of continuous sheets 92CS, 94CS while continuing to exhibit elasticity is considered active in elasticity. Such area is considered an elastic region. Some of the first group of elastic bodies 96FC may be removed of its elasticity in regions planned to be joined to the transverse center of the central chassis 38, and then deactivating its elasticity to form a non-elastic region 22. Removal of elasticity from a certain area of the elastic belt 40 overlapping with the corresponding front and/or back waist panel 52, 54 may be advantageous when the center chassis 38 comprises an absorbent material existing region 62, in that elasticity in the front and/or back waist panel 52, 54 may cause bunching of the absorbent material existing region 62 and interfere with close fit of the center chassis 38 to the wearer.

Referring to Figures 8 and 9, the repetition of the elastic region and non-elastic region 22 on the FEP may be made by intermittently joining the first and second layers of continuous sheets 92CS, 94CS, followed by deactivating the elastic bodies disposed in the unjoined regions. The elasticity of the first group of elastic bodies 96FC may be removed by either leaving the elastic body unadhered to the first and second layers of continuous sheets 92CS, 94CS or adhering them to one or both of the first and second layers of continuous sheets 92CS, 94CS with significantly weaker adhesion than in the elastic region, and then deactivating the elastic bodies disposed in the unadhered or weakly adhered portion. A cutting unit may be employed for effective deactivation, and may be configured with a die knife, flexible blade, and/or compression roll features, and may also include additional features to control knife-anvil gaps and/or force, and may include means for heating. The cutting unit may include a cutting roll 600, an anvil roll 602, and a flexible blade 612 extending from the roll circumference surface 606 of the cutting roll 600.

All of the first group of elastic bodies 96FC may have a non-elastic region 22. Only a portion of the first group of elastic bodies 96FC may be provided with non-elastic regions 22, for example, those that are planned to be disposed closer to the proximal edge 90 of the resulting article. Accordingly, some of the first group of elastic bodies 96FC may be configured to remain elastic across the planned width of the elastic belt 40. The machine direction dimension of the non-elastic region 22 per elastic body may be varied.

### Step B)

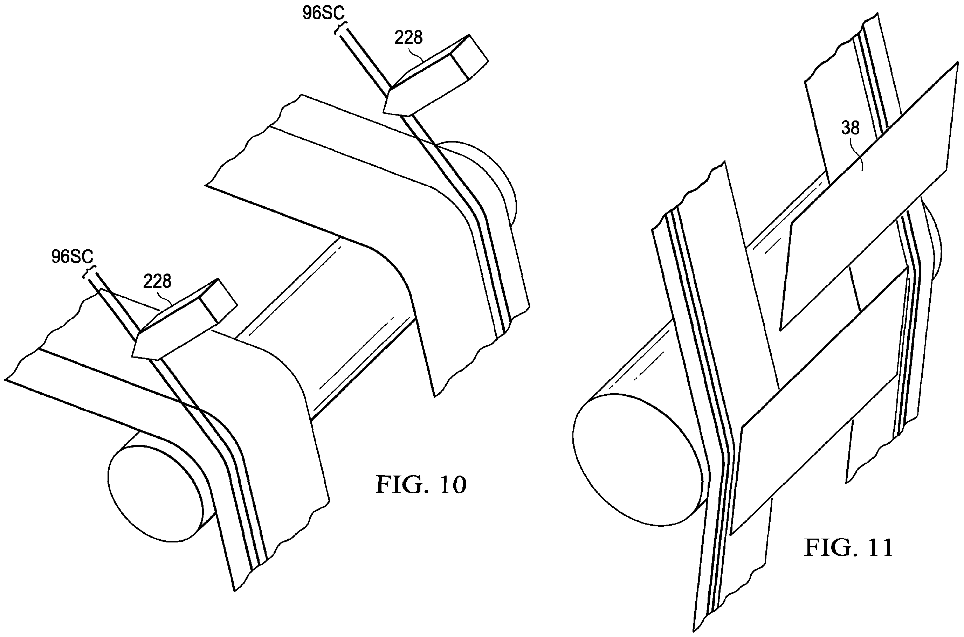

Referring to Figures 8 and 10, the thus obtained PEBP is then sent to introduce the second group of elastic bodies 96SC comprising the steps of: advancing a second group of elastic bodies 96SC in the machine direction in a stretched state and joining the second group of elastic bodies 96SC to the first surface of the first layer of continuous sheet 92CS of the PEBP. The portion at which the second group of elastic bodies 96SC are joined may be referred to as the second elastic portion, or SEP. The SEP may be configured to be elastic across the planned width of the elastic belt 40.

### Step C)

Referring to Figures 8 and 11, the thus obtained assembly is then sent to joining with the central chassis 38. The joining of the central chassis 38 may be provided by applying adhesive where the central chassis 38 is planned to be positioned. Adhesive may be provided in the entire region where the central chassis 38 overlaps, or in intervals. The positioning of the second group of elastic bodies 96SC may be selected such that the elastic body closest to the proximal edge of the first layer fold over of continuous sheet 93CS is disposed at a distance D6 away from the proximal edge of the first layer fold over of continuous sheet 93CS, wherein D6 may be at least about 5mm. Referring to Figures 5-6, this may prevent any elastic body 96 from being accidentally left uncovered by the outer sheet fold over 93 in the finished article.

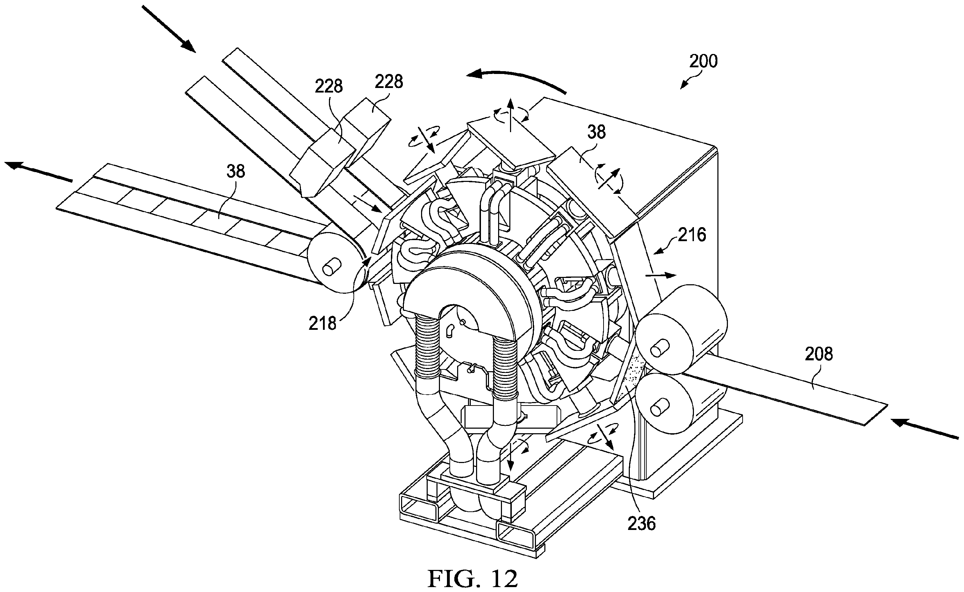

Preceding this step, referring to Figure 12, the central chassis 38 may be formed by cutting a continuous central chassis 208 into discrete pieces, and processing through a transfer assembly 200. The transfer surface 236 of the transfer assembly 200 may pick up the central chassis 38 in a first position 216, turn the transfer surface 236 together with the central chassis 38 into a second position 218 while spacing the central chassis 38 as appropriate, and transferred to be joined such that the transverse axis of the central chassis 38 is aligned with the machine direction of the elastic belt making assembly. During the turning, the central chassis 38 may be held by the transfer surface 236 via vacuum. Upon transferring of the central chassis 38 from the transfer surface 236 to the assembly, vaccum is released, and instead the central chassis 38 is pressed onto the assembly for securely joining. In this assembling step, to avoid any transfer of adhesive glue or any other undesirable material to the transfer surface 236 from the central chassis 38 overlapping region of the assembly or from the second group of elastic bodies 96SC in the SEP, the second layer of continuous sheet 94CS may be planned to have a wider dimension in the cross machine direction than that of the central chassis 38 by more than 0mm to no more than about 30mm. Namely, in the resulting article, the distal edge of the second layer of continuous sheet which becomes the proximal edge of the inner sheet 94PE is distal than the distal edge of the central chassis 38DE as in Figures 3A-B. Alternatively, in the resulting article, the distal edge of the central chassis 38DE may be distal than the distal edge of the second layer of continuous sheet which becomes the proximal edge of the inner sheet 94PE as in Figures 5-7. For making the articles of Figures 5-7, joining of the central chassis 38 is carefully registered with the assembly in both the machine direction and the cross machine direction to avoid transfer of adhesive glue or any other undesirable material to the transfer surface 236.

### Step D)

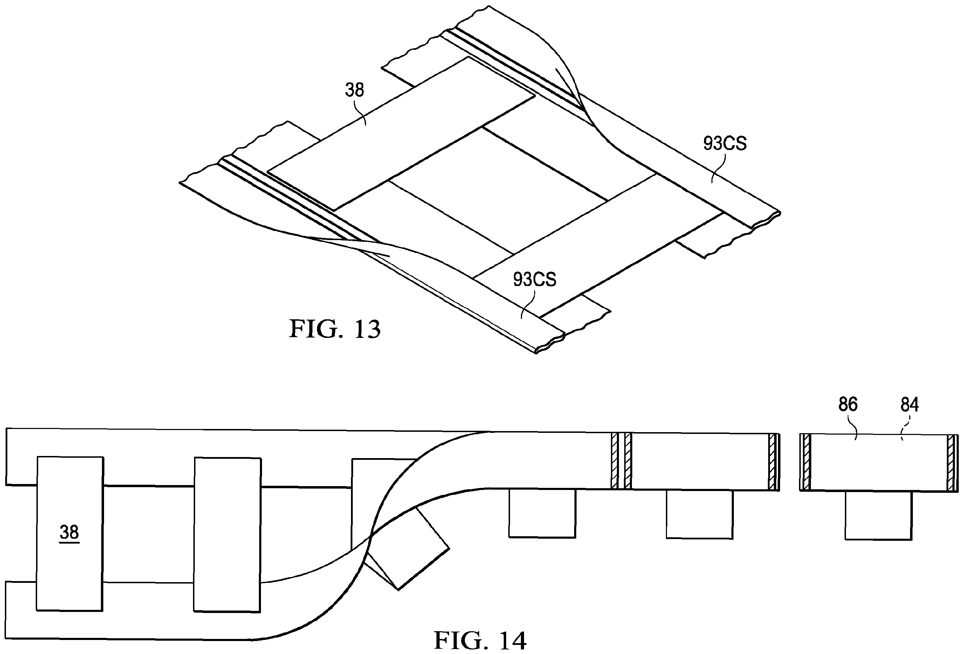

Referring to Figure 13, the obtained assembly is then sent to folding the first layer to form a first layer fold over of continuous sheet 93CS, such that at least one of the second group of elastic bodies 96SC is directly joined between the first surface of the first layer of continuous sheets 92CS and the first surface of the first layer fold over of continuous sheets 93CS.

As explained above, the second layer of continuous sheet 94CS may be planned to have a wider dimension in the cross machine direction than that of the central chassis 38. Further, the first layer fold over of continuous sheet 93CS may extend to overlap the central chassis 38. Referring to Figures 3A-B and 5-7, by taking such configuration in the SEP, the resulting article has the center chassis 38 securely sandwiched by at least the inner sheet 94 and the outer sheet fold over 93 in the vicinity of the longitudinal edge 38E by having an overlap D3 with the outer sheet fold over 93 in the longtitudinal direction, wherein D3 may be from about 10 mm to about 50mm, or from about 10 mm to about 45mm. Referring to Figures 3A-B and 5-7, in the resulting article, the inner sheet 94, however, does not extend to the distal edge 88 of the belt, thus, leaving some region where the belt is configured only by the outer sheet 92 and the outer sheet fold over 93. The inner belt distal edge 94DE may have a distance D4 from the belt distal edge 88, wherein D4 may be from about 10mm to about 45mm. By having such distance, meaningful cost saving of the inner sheet 94 is provided, while maintaining overall rigidity for the article. There may be at least 2 second group of elastic bodies 96SC, or from about 2 to about 8 second group of elastic bodies 96SC, or from about 2 to about 6 second group of elastic bodies per one side of the belt.

Further, referring to Figures 3C and 9, there may be a stiff region 37 in the FEP which is positioned in the resulting article towards the inner sheet distal edge 94DE wherein the outer sheet fold over 93, inner sheet 94, and outer sheet 92 overlap in the thickness direction. The stiff region 37 may overlap the absorbing material non-existing region 61 of the central chassis. By having many layers in this stiff region 37, rigidity and stiffness is provided to prevent bending. There is advantage to provide such stiffness in the stiff region 37, as this region tends to bend and thus may cause leakage during wear. For balancing the stiffness provided by this region while minimizing overlap of materials, the stiff region 37 is disposed of one to 4 elastics 96, or one to two elastics 96, and may have a longitudinal dimension of from about 6mm to about 40mm, or from about 6mm to about 30mm.

Referring to Figure 14, the thus obtained assembly is then folded at the middle point in the cross machine direction, seamed at the edges of the planned edges of the article, and then finally cut to obtain a wearable article.

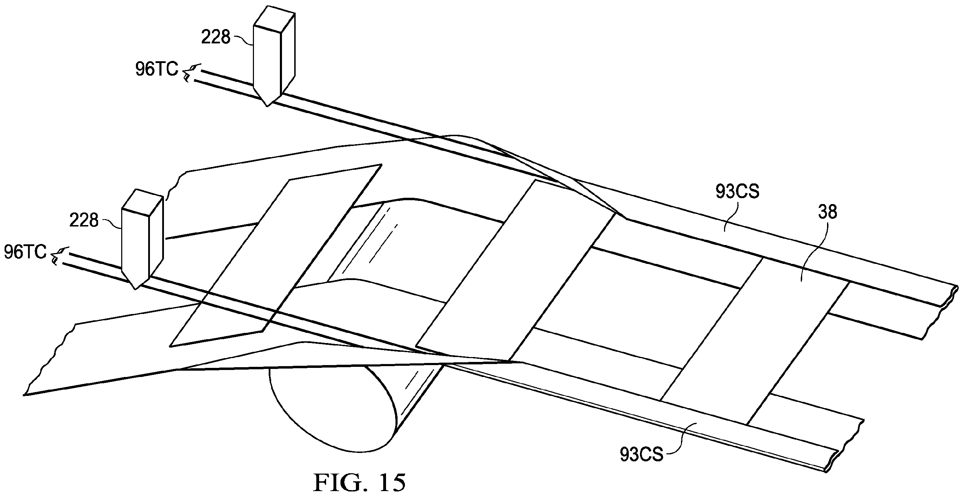

Referring to Figure 15, the present method may further comprise the steps, prior to Step D) of:
advancing the assembly in a machine direction;
advancing a third group of elastic bodies 96TC in the machine direction in a stretched state;
and then joining the third group of elastic bodies 96TC between the central chassis 38 and the first layer fold over of continuous sheet 93CS. The third group of elastic bodies may be introduced, for example, to overlap with the stiff region 37, and in a continuously elastic manner. Such elastic bodies may contribute to providing stiffness in the region to prevent bending in lieu of the second group of elastic bodies 96SC. Disposing elastic bodies closer to the wearer may be advantageous to provide closer fit.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A method of manufacturing a wearable article (20) assembled by a front belt (84), a back belt (86), and a central chassis (38), the central chassis (38) having a front waist region (52) and a back waist region (54) separated from each other by a crotch region (56); wherein at least one of the front belt (84) and the back belt (86) is formed by an elastic belt,
the method comprising making the elastic belt (84. 86) and joining to the central chassis (38) by the steps of;
advancing a first layer of continuous sheet (92CS) having a first surface and an opposing second surface in a machine direction and defining a width in a cross machine direction;
advancing a second layer of continuous sheet (94CS) having a first surface and an opposing second surface in the machine direction and having a smaller width than the first layer of continuous sheet (92CS);
advancing a first group of elastic bodies (96FC) in the machine direction in a stretched state; joining the first group of elastic bodies between the first surface of the first layer and the first surface of the second layer of continuous sheets (92CS, 94CS) to form a primary elastic belt precursor
advancing a second group of elastic bodies (96SC) in the machine direction in a stretched state; joining the second group of elastic bodies (96SC) to the first surface of the first layer of continuous sheet (92CS) of the primary elastic belt precursor;
joining the central chassis (38) to the second surface of the second layer of continuous sheet (94CS);
folding the first layer of continuous sheet to form a first layer fold over of continuous sheet (93CS), such that at least one of the second group of elastic bodies (96SC) is directly joined between the first surface of the first layer of continuous sheet (92CS) and the first surface of the first layer fold over of continuous sheet (93CS).

2. The method of any of the preceding claims further comprising the steps of:
advancing a third group of elastic bodies (96TC) in the machine direction in a stretched state; and
joining the third group of elastic bodies (96TC) between the central chassis (38) and the first layer fold over of continuous sheet (93CS).

3. The method of Claim 1 or 2 wherein the distal edge of the central chassis (38) is distal than the distal edge of the second layer of continuous sheet (94).

4. A method of manufacturing a wearable article (20) assembled by a front belt (84), a back belt (86), and a central chassis (38), the central chassis (38) having a front waist region (52) and a back waist region (54) separated from each other by a crotch region (56); wherein at least one of the front belt (84) and the back belt (84) is formed by an elastic belt,
the method comprising making the elastic belt (84, 86) and joining to the central chassis (38) by the steps of;
advancing a first layer of continuous sheet (92CS) having a first surface and an opposing second surface in a machine direction and defining a width in a cross machine direction;
advancing a second layer of continuous sheet (94CS) having a first surface and an opposing second surface in the machine direction and having a smaller width than the first layer of continuous sheet (92CS);
advancing a first group of elastic bodies (96FC) in the machine direction in a stretched state; joining the first group of elastic bodies (96FC) between the first surface of the first layer and the first surface of the second layer of continuous sheets (92CS, 94CS) to form a primary elastic belt precursor;
joining the central chassis (38) to the second surface of the second layer of continuous sheet (94CS) of the primary elastic belt precursor;
advancing a second group of elastic bodies (96SC) in the machine direction in a stretched state; joining the second group of elastic bodies (96SC) to the first surface of the first layer of continuous sheet (92CS);
folding the first layer of continuous sheet to form a first layer fold over of continuous sheet (93CS), such that at least one of the second group of elastic bodies (96SC) is directly joined between the first surface of the first layer of continuous sheet (92CS) and the first surface of the first layer fold over of continuous sheet (93CS).

5. The method of any of the preceding claims wherein at least a portion of the central chassis (38) is joined between the first layer fold over (93CS) and the second surface of the second layer of continuous sheets (92CS).

6. The method of any of the preceding claims wherein the distal edge of the second layer of continuous sheet (92CS) is distal than the distal edge of the central chassis (38).

7. The method of any of the preceding claims further comprising the step of:
dividing the primary elastic belt precursor by a line along the machine direction to form a front primary elastic belt precursor and a back primary elastic belt precursor prior to joining with the second group of elastic bodies (96SC).

8. The method of Claim 7 wherein the front primary elastic belt precursor and the back primary elastic belt precursor are different in at least one of; the dimension of the precursor, the number of first group of elastic bodies (96FC), and the positioning of the first group of elastic bodies (96FC).

9. The method of any of the preceding claims wherein the primary elastic belt precursor comprises an elastic region and a non-elastic region intermittently spaced apart in the machine direction.

10. The method of Claim 9 wherein the non-elastic region is formed by leaving the first group of elastic bodies (96FC) disposed on the non-elastic region unjoined to the adjacent sheets or joined with lower adhesion compared to the remaining joined regions, followed by deactivating the elastic bodies (96FC) disposed on the non-elastic region.

11. A method of manufacturing a wearable article (20) assembled by a front belt (84), a back belt (86), and a central chassis (38), the central chassis (38) having a front waist region (52) and a back waist region (54) separated from each other by a crotch region (56);
the method comprising making the front belt (84) and the back belt (86) and joining to the central chassis (38) by the steps of;
advancing a first layer of continuous sheet (92CS) having a first surface and an opposing second surface in a machine direction and defining a width in a cross machine direction;
advancing a second layer of continuous sheet (94CS) having a first surface and an opposing second surface in the machine direction and having a smaller width than the first layer of continuous sheet (92CS);
advancing a first group of elastic bodies (96FC) in the machine direction in a stretched state;
joining the first group of elastic bodies (96FC) between the first surface of the first layer and the first surface of the second layers of continuous sheets (94CS) to form a primary elastic belt precursor, the primary elastic belt precursor comprising a non-elastic region formed by leaving a portion of the elastic bodies (96FC) disposed on the non-elastic region unjoined to the first and second layers of continuous sheets (92CS, 94CS);
dividing the primary elastic belt precursor by a line continuous in the machine direction to obtain a continuous front elastic belt percursor and a continuous back elastic belt percursor and deactivating the elastic bodies (96FC) disposed on the non-elastic region;
advancing the continuous front elastic belt precursor and the continuous back elastic belt precursor in the machine direction;
advancing two sets of a second group of elastic bodies (96SC) in the machine direction in a stretched state;
joining the second group of elastic bodies (96SC) on the first surface of the first layer of each of the continuous front elastic belt precursor and the continuous back elastic belt percursor;
joining the front waist region (52) of the central chassis (38) to the non-elastic region of the continuous front elastic belt precursor and joining the back waist region (54) of the central chassis (38) to the non-elastic region of the continuous back elastic belt percursor;
folding the first layer of continuous sheets of each of the continuous front elastic belt precursor and the continuous back elastic belt precursor, to form a first layer fold over of continuous sheets (93CS), such that at least one of the second group of elastic bodies (96SC) is directly joined between the first surface of the first layer of continuous sheets (92CS) and the first surface of the first layer fold over of continuous sheets (93CS).

## Patentansprüche

1. Verfahren zum Fertigen eines tragbaren Gegenstands (20), der durch ein vorderseitiges Band (84), ein rückseitiges Band (86) und eine mittlere Grundeinheit (38) zusammengesetzt wird, wobei die mittlere Grundeinheit (38) einen vorderseitigen Taillenbereich (52) und einen rückseitigen Taillenbereich (54) aufweist, die durch einen Schrittbereich (56) voneinander getrennt sind; wobei mindestens eins von dem vorderseitigen Band (84) und dem rückseitigen Band (86) durch ein elastisches Band gebildet wird,
wobei das Verfahren das Herstellen des elastischen Bands (84. 86) und das Verbinden mit der mittleren Grundeinheit (38) umfasst durch die Schritte des;
Vorwärtsbewegens einer ersten Schicht aus ununterbrochener Lage (92CS), die in einer Maschinenrichtung eine erste Oberfläche und eine gegenüberliegende zweite Oberfläche aufweist und die eine Breite in einer Maschinenquerrichtung definiert;
Vorwärtsbewegens einer zweiten Schicht aus ununterbrochener Lage (94CS), die in der Maschinenrichtung eine erste Oberfläche und eine gegenüberliegende zweite Oberfläche aufweist und eine kleinere Breite als die erste Schicht aus ununterbrochener Lage (92CS) aufweist;
Vorwärtsbewegens einer ersten Gruppe von elastischen Körpern (96FC) in der Maschinenrichtung in einem gedehnten Zustand;
Verbindens der ersten Gruppe von elastischen Körpern zwischen der ersten Oberfläche der ersten Schicht und der ersten Oberfläche der zweiten Schicht aus ununterbrochenen Lagen (92CS, 94CS), um einen primären elastischen Bandvorläufer zu bilden
Vorwärtsbewegens einer zweiten Gruppe von elastischen Körpern (96SC) in der Maschinenrichtung in einem gedehnten Zustand;
Verbindens der zweiten Gruppe von elastischen Körpern (96SC) mit der ersten Oberfläche der ersten Schicht aus ununterbrochener Lage (92CS) des primären elastischen Bandvorläufers;
Verbindens der mittleren Grundeinheit (38) mit der zweiten Oberfläche der zweiten Schicht aus ununterbrochener Lage (94CS);
Faltens der ersten Schicht aus ununterbrochener Lage, um eine erste Schichtfaltung über aus ununterbrochener Lage (93CS) zu bilden, sodass mindestens einer von der zweiten Gruppe von elastischen Körpern (96SC) direkt zwischen der ersten Oberfläche der ersten Schicht aus ununterbrochener Lage (92CS) und der ersten Oberfläche der ersten Schichtfaltung über aus ununterbrochener Lage (93CS) verbunden wird.

2. Verfahren nach einem der vorstehenden Ansprüche, ferner umfassend die Schritte des:
Vorwärtsbewegens einer dritten Gruppe von elastischen Körpern (96TC) in der Maschinenrichtung in einem gedehnten Zustand; und
Verbindens der dritten Gruppe von elastischen Körpern (96TC) zwischen der mittleren Grundeinheit (38) und der ersten Schichtfaltung über aus ununterbrochener Lage (93CS).

3. Verfahren nach Anspruch 1 oder 2, wobei der distale Rand der mittleren Grundeinheit (38) distal ist als der distale Rand der zweiten Schicht aus ununterbrochener Lage (94).

4. Verfahren zum Fertigen eines tragbaren Gegenstands (20), der durch ein vorderseitiges Band (84), ein rückseitiges Band (86) und eine mittlere Grundeinheit (38) zusammengesetzt wird, wobei die mittlere Grundeinheit (38) einen vorderseitigen Taillenbereich (52) und einen rückseitigen Taillenbereich (54) aufweist, die durch einen Schrittbereich (56) voneinander getrennt sind; wobei mindestens eins von dem vorderseitigen Band (84) und dem rückseitigen Band (84) durch ein elastisches Band gebildet wird,
wobei das Verfahren das Herstellen des elastischen Bands (84, 86) und das Verbinden mit der mittleren Grundeinheit (38) umfasst durch die Schritte des;
Vorwärtsbewegens einer ersten Schicht aus ununterbrochener Lage (92CS), die in einer Maschinenrichtung eine erste Oberfläche und eine gegenüberliegende zweite Oberfläche aufweist und die eine Breite in einer Maschinenquerrichtung definiert;
Vorwärtsbewegens einer zweiten Schicht aus ununterbrochener Lage (94CS), die in der Maschinenrichtung eine erste Oberfläche und eine gegenüberliegende zweite Oberfläche aufweist und eine kleinere Breite als die erste Schicht aus ununterbrochener Lage (92CS) aufweist;
Vorwärtsbewegens einer ersten Gruppe von elastischen Körpern (96FC) in der Maschinenrichtung in einem gedehnten Zustand;
Verbindens der ersten Gruppe von elastischen Körpern (96FC) zwischen der ersten Oberfläche der ersten Schicht und der ersten Oberfläche der zweiten Schicht aus ununterbrochenen Lagen (92CS, 94CS), um einen primären elastischen Bandvorläufer zu bilden;
Verbindens der mittleren Grundeinheit (38) mit der zweiten Oberfläche der zweiten Schicht aus ununterbrochener Lage (94CS) des primären elastischen Bandvorläufers;
Vorwärtsbewegens einer zweiten Gruppe von elastischen Körpern (96SC) in der Maschinenrichtung in einem gedehnten Zustand;
Verbindens der zweiten Gruppe von elastischen Körpern (96SC) mit der ersten Oberfläche der ersten Schicht aus ununterbrochener Lage (92CS);
Faltens der ersten Schicht aus ununterbrochener Lage, um eine erste Schichtfaltung über aus ununterbrochener Lage (93CS) zu bilden, sodass mindestens einer von der zweiten Gruppe von elastischen Körpern (96SC) direkt zwischen der ersten Oberfläche der ersten Schicht aus ununterbrochener Lage (92CS) und der ersten Oberfläche der ersten Schichtfaltung über aus ununterbrochener Lage (93CS) verbunden wird.

5. Verfahren nach einem der vorstehenden Ansprüche, wobei zumindest ein Abschnitt der mittleren Grundeinheit (38) zwischen der ersten Schichtfaltung über (93CS) und der zweiten Oberfläche der zweiten Schicht ununterbrochener Lagen (92CS) verbunden wird.

6. Verfahren nach einem der vorstehenden Ansprüche, wobei der distale Rand der zweiten Schicht aus ununterbrochener Lage (92CS) distal ist als der distale Rand der mittleren Grundeinheit (38).

7. Verfahren nach einem der vorstehenden Ansprüche, ferner umfassend den Schritt des:
Teilens des primären elastischen Bandvorläufers durch eine Linie entlang der Maschinenrichtung, um vor dem Verbinden mit der zweiten Gruppe von elastischen Körpern (96SC) einen vorderseitigen primären elastischen Bandvorläufer und einen rückseitigen primären elastischen Bandvorläufer zu bilden.

8. Verfahren nach Anspruch 7, wobei sich der vorderseitige primäre elastische Bandvorläufer und der rückseitige primäre elastische Bandvorläufer unterscheiden hinsichtlich mindestens einem von; der Abmessung des Vorläufers, der Anzahl der ersten Gruppe von elastischen Körpern (96FC) und der Positionierung der ersten Gruppe von elastischen Körpern (96FC).

9. Verfahren nach einem der vorstehenden Ansprüche, wobei der primäre elastische Bandvorläufer einen elastischen Bereich und einen nichtelastischen Bereich umfasst, die in der Maschinenrichtung intermittierend beabstandet sind.

10. Verfahren nach Anspruch 9, wobei der nichtelastische Bereich gebildet wird, indem die erste Gruppe von elastischen Körpern (96FC), die auf dem nichtelastischen Bereich angeordnet sind, mit den benachbarten Lagen unverbunden oder mit einer geringeren Haftung im Vergleich zu den restlichen verbundenen Bereichen verbunden belassen wird, gefolgt von Deaktivieren der elastischen Körper (96FC), die auf dem nichtelastischen Bereich angeordnet sind.

11. Verfahren zum Fertigen eines tragbaren Gegenstands (20), der durch ein vorderseitiges Band (84), ein rückseitiges Band (86) und eine mittlere Grundeinheit (38) zusammengesetzt wird, wobei die mittlere Grundeinheit (38) einen vorderseitigen Taillenbereich (52) und einen rückseitigen Taillenbereich (54) aufweist, die durch einen Schrittbereich (56) voneinander getrennt sind;
wobei das Verfahren das Herstellen des vorderseitigen Bands (84) und des rückseitigen Bands (86) und das Verbinden mit der mittleren Grundeinheit (38) umfasst durch die Schritte des;
Vorwärtsbewegens einer ersten Schicht aus ununterbrochener Lage (92CS), die in einer Maschinenrichtung eine erste Oberfläche und eine gegenüberliegende zweite Oberfläche aufweist und die eine Breite in einer Maschinenquerrichtung definiert;
Vorwärtsbewegens einer zweiten Schicht aus ununterbrochener Lage (94CS), die in der Maschinenrichtung eine erste Oberfläche und eine gegenüberliegende zweite Oberfläche aufweist und eine kleinere Breite als die erste Schicht aus ununterbrochener Lage (92CS) aufweist;
Vorwärtsbewegens einer ersten Gruppe von elastischen Körpern (96FC) in der Maschinenrichtung in einem gedehnten Zustand;
Verbindens der ersten Gruppe von elastischen Körpern (96FC) zwischen der ersten Oberfläche der ersten Schicht und der ersten Oberfläche der zweiten Schichten aus ununterbrochenen Lagen (94CS), um einen primären elastischen Bandvorläufer zu bilden, wobei der primäre elastische Bandvorläufer einen nichtelastischen Bereich umfasst, der gebildet wird, indem ein Abschnitt der elastischen Körper (96FC), die auf dem nichtelastischen Bereich angeordnet sind, unverbunden mit der ersten und der zweiten Schicht aus ununterbrochenen Lagen (92CS, 94CS) belassen wird;
Teilens des primären elastischen Bandvorläufers durch eine in der Maschinenrichtung ununterbrochene Linie, um einen ununterbrochenen vorderseitigen elastischen Bandvorläufer und einen ununterbrochenen rückseitigen elastischen Bandvorläufer zu bilden, und Deaktivierens der elastischen Körper (96FC), die auf dem nichtelastischen Bereich angeordnet sind;
Vorwärtsbewegens des ununterbrochenen vorderseitigen elastischen Bandvorläufers und des ununterbrochenen rückseitigen elastischen Bandvorläufers in der Maschinenrichtung;
Vorwärtsbewegens von zwei Sätzen einer zweiten Gruppe von elastischen Körpern (96SC) in der Maschinenrichtung in einem gedehnten Zustand;
Verbindens der zweiten Gruppe von elastischen Körpern (96SC) auf der ersten Oberfläche der ersten Schicht von jedem der ununterbrochenen vorderseitigen elastischen Bandvorläufer und des ununterbrochenen rückseitigen elastischen Bandvorläufers;
Verbindens des vorderseitigen Taillenbereichs (52) der mittleren Grundeinheit (38) mit dem nichtelastischen Bereich des ununterbrochenen vorderseitigen elastischen Bandvorläufers und Verbindens des rückseitigen Taillenbereichs (54) der mittleren Grundeinheit (38) mit dem nichtelastischen Bereich des ununterbrochenen rückseitigen elastischen Bandvorläufers;
Faltens der ersten Schicht aus ununterbrochenen Lagen von jedem des ununterbrochenen vorderseitigen elastischen Bandvorläufers und des ununterbrochenen rückseitigen elastischen Bandvorläufers, um eine erste Schichtfaltung über aus ununterbrochenen Lagen (93CS) zu bilden, sodass mindestens einer von der zweiten Gruppe von elastischen Körpern (96SC) direkt zwischen der ersten Oberfläche der ersten Schicht aus ununterbrochenen Lagen (92CS) und der ersten Oberfläche der ersten Schichtfaltung über aus ununterbrochenen Lagen (93CS) verbunden wird.

## Revendications

1. Procédé de fabrication d'un article portable (20) assemblé par une courroie avant (84), une courroie arrière (86), et un châssis central (38), le châssis central (38) ayant une région de ceinture avant (52) et une région de ceinture arrière (54) séparées l'une de l'autre par une région d'entrejambe (56) ; dans lequel au moins l'une parmi la courroie avant (84) et la courroie arrière (86) est formée d'une courroie élastique,
le procédé comprenant la réalisation de la courroie élastique (84. 86) et le raccordement au châssis central (38) par les étapes consistant à ;
faire avancer une première couche de feuille continue (92CS) ayant une première surface et une deuxième surface opposée dans un sens machine et définissant une largeur dans un sens machine transversal ;
faire avancer une deuxième couche de feuille continue (94CS) ayant une première surface et une deuxième surface opposée dans le sens machine et ayant une largeur plus petite que la première couche de feuille continue (92CS) ;
faire avancer un premier groupe de corps élastiques (96FC) dans le sens machine dans un état allongé ;
raccorder le premier groupe de corps élastiques entre la première surface de la première couche et la première surface de la deuxième couche de feuilles continues (92CS, 94CS) pour former un précurseur de courroie élastique primaire
faire avancer un deuxième groupe de corps élastiques (96SC) dans le sens machine dans un état allongé ;
raccorder le deuxième groupe de corps élastiques (96SC) à la première surface de la première couche de feuille continue (92CS) du précurseur de courroie élastique primaire ;
raccorder le châssis central (38) à la deuxième surface de la deuxième couche de feuille continue (94CS) ;
plier la première couche de feuille continue pour former une première couche pliée de feuille continue (93CS), de telle sorte qu'au moins l'un du deuxième groupe de corps élastiques (96SC) soit directement raccordé entre la première surface de la première couche de feuille continue (92CS) et la première surface de la première couche pliée de feuille continue (93CS).

2. Procédé selon l'une quelconque des revendications précédentes comprenant en outre les étapes consistant à :
faire avancer un troisième groupe de corps élastiques (96TC) dans le sens machine dans un état allongé ; et
raccorder le troisième groupe de corps élastiques (96TC) entre le châssis central (38) et la première couche pliée de feuille continue (93CS).

3. Procédé selon la revendication 1 ou 2, dans lequel le bord distal du châssis central (38) est distal que le bord distal de la deuxième couche de feuille continue (94).

4. Procédé de fabrication d'un article portable (20) assemblé par une courroie avant (84), une courroie arrière (86), et un châssis central (38), le châssis central (38) ayant une région de ceinture avant (52) et une région de ceinture arrière (54) séparées l'une de l'autre par une région d'entrejambe (56) ; dans lequel au moins l'une parmi la courroie avant (84) et la courroie arrière (84) est formée d'une courroie élastique,
le procédé comprenant la réalisation de la courroie élastique (84, 86) et le raccordement au châssis central (38) par les étapes consistant à ;
faire avancer une première couche de feuille continue (92CS) ayant une première surface et une deuxième surface opposée dans un sens machine et définissant une largeur dans un sens machine transversal ;
faire avancer une deuxième couche de feuille continue (94CS) ayant une première surface et une deuxième surface opposée dans le sens machine et ayant une largeur plus petite que la première couche de feuille continue (92CS) ;
faire avancer un premier groupe de corps élastiques (96FC) dans le sens machine dans un état allongé ;
raccorder le premier groupe de corps élastiques (96FC) entre la première surface de la première couche et la première surface de la deuxième couche de feuilles continues (92CS, 94CS) pour former un précurseur de courroie élastique primaire ;
raccorder le châssis central (38) à la deuxième surface de la deuxième couche de feuille continue (94CS) du précurseur de courroie élastique primaire ;
faire avancer un deuxième groupe de corps élastiques (96SC) dans le sens machine dans un état allongé ;
raccorder le deuxième groupe de corps élastiques (96SC) à la première surface de la première couche de feuille continue (92CS) ;
plier la première couche de feuille continue pour former une première couche pliée de feuille continue (93CS), de telle sorte qu'au moins l'un du deuxième groupe de corps élastiques (96SC) soit directement raccordé entre la première surface de la première couche de feuille continue (92CS) et la première surface de la première couche pliée de feuille continue (93CS).

5. Procédé selon l'une quelconque des revendications précédentes dans lequel au moins une partie du châssis central (38) est raccordée entre la première couche pliée (93CS) et la deuxième surface de la deuxième couche de feuilles continues (92CS).

6. Procédé selon l'une quelconque des revendications précédentes dans lequel le bord distal de la deuxième couche de feuille continue (92CS) est distal que le bord distal du châssis central (38).

7. Procédé selon l'une quelconque des revendications précédentes comprenant en outre l'étape consistant à :
diviser le précurseur de courroie élastique primaire par une ligne le long du sens machine pour former un précurseur de courroie élastique primaire avant et un précurseur de courroie élastique primaire arrière antérieurement au raccordement avec le deuxième groupe de corps élastiques (96SC).

8. Procédé selon la revendication 7 dans lequel le précurseur de courroie élastique primaire avant et le précurseur de courroie élastique primaire arrière sont différents dans au moins l'un parmi ; la dimension du précurseur, le nombre de premier groupe de corps élastiques (96FC), et le positionnement du premier groupe de corps élastiques (96FC).

9. Procédé selon l'une quelconque des revendications précédentes dans lequel le précurseur de courroie élastique primaire comprend une région élastique et une région non élastique espacées de façon intermittente dans le sens machine.

10. Procédé selon la revendication 9 dans lequel la région non élastique est formée en laissant le premier groupe de corps élastiques (96FC) disposés sur la région non élastique non raccordée aux feuilles adjacentes ou raccordée avec une adhérence inférieure par comparaison avec les régions raccordées restantes, puis en désactivant les corps élastiques (96FC) disposés sur la région non élastique.

11. Procédé de fabrication d'un article portable (20) assemblé par une courroie avant (84), une courroie arrière (86), et un châssis central (38), le châssis central (38) ayant une région de ceinture avant (52) et une région de ceinture arrière (54) séparées l'une de l'autre par une région d'entrejambe (56) ;
le procédé comprenant la réalisation de la courroie avant (84) et de la courroie arrière (86) et le raccordement au châssis central (38) par les étapes consistant à ;
faire avancer une première couche de feuille continue (92CS) ayant une première surface et une deuxième surface opposée dans un sens machine et définissant une largeur dans un sens machine transversal ;
faire avancer une deuxième couche de feuille continue (94CS) ayant une première surface et une deuxième surface opposée dans le sens machine et ayant une largeur plus petite que la première couche de feuille continue (92CS) ;
faire avancer un premier groupe de corps élastiques (96FC) dans le sens machine dans un état allongé ;
le raccordement du premier groupe de corps élastiques (96FC) entre la première surface de la première couche et la première surface des deuxièmes couches de feuilles continues (94CS) pour former un précurseur de courroie élastique primaire, le précurseur de courroie élastique primaire comprenant une région non élastique formée en laissant une partie des corps élastiques (96FC) disposés sur la région non élastique non raccordée aux première et deuxième couches de feuilles continues (92CS, 94CS) ;
diviser le précurseur de courroie élastique primaire par une ligne continue dans le sens machine pour obtenir un précurseur de courroie élastique avant continu et un précurseur de courroie élastique arrière continu et désactiver les corps élastiques (96FC) disposés sur la région non élastique ;
faire avancer le précurseur de courroie élastique avant continu et le précurseur de courroie élastique arrière continu dans le sens machine ;
faire avancer deux ensembles d'un deuxième groupe de corps élastiques (96SC) dans le sens machine dans un état allongé ;
raccorder le deuxième groupe de corps élastiques (96SC) sur la première surface de la première couche de chacun du précurseur de courroie élastique avant continu et du précurseur de courroie élastique arrière continu ;
raccorder la région de ceinture avant (52) du châssis central (38) à la région non élastique du précurseur de courroie élastique avant continu et raccorder la région de ceinture arrière (54) du châssis central (38) à la région non élastique du précurseur de courroie élastique arrière continu ;
plier la première couche de feuilles continues de chacun du précurseur de courroie élastique avant continu et du précurseur de courroie élastique arrière continu, pour former une première couche pliée de feuilles continues (93CS), de telle sorte qu'au moins l'un du deuxième groupe de corps élastiques (96SC) soit directement raccordé entre la première surface de la première couche de feuilles continues (92CS) et la première surface de la première couche pliée de feuilles continues (93CS).
